# EUROPEAN PATENT SPECIFICATION

(11) **EP 1 687 393 B1**
(45) Date of publication and mention of the grant of the patent: **07.03.2012**
(21) Application number: 04818801.5
(22) Date of filing: 18.11.2004
(51) Int. Cl.: C12M 1/00, C12M 1/04

(54) **SYSTEM FOR CELL CULTURE**
SYSTEM FÜR DIE ZELLKULTUR
SYSTEME DE CULTURE CELLULAIRE

(30) Priority: 18.11.2003 EP 03026391
(43) Date of publication of application: 09.08.2006
(73) Proprietor: Nestec S.A., 1800 Vevey (CH)
(72) Inventor: COURTOIS Didier, F-37550 St. Avertin (FR); CUVIER, Arnaud, Robert, Gilles, F-37170 Chambray-les-Tours (FR); HENAULT, Nicolas, F-51120 Sezanne (FR)
(86) International application number: PCT/EP2004/013083
(87) International publication number: WO 2005/049784

(56) References cited:
- WO-A-00/66706
- FR-A- 2 519 020

## Description

### Field of the invention

The present invention relates to the field of cell culture. It consists in a liquid culture system, for growing cells in general and plant cells in particular. This culture system is based on the principle of a wave providing agitation, which offers a convenient mixing, and aeration to the cell culture contained into a disposable plastic bag. Such a large-scale, efficient and disposable culture system can largely reduce production costs.

### Background of the invention

Conventional culture systems are generally composed of a rigid container (glass or stainless steel) having means for aerating and mixing the culture content (air sparger, impeller). These systems are complex, and usual equipment and support facilities associated with aseptic bioprocess are extremely expensive because the large-scale production is based on stainless steel vessels, sterilized *in situ.* More than 60% of the production costs is due to the fixed costs : high capital costs of fermentation equipment, depreciation, interest and capital expenditure. The running costs are also high, due to low yields and the needs to clean and sterilize the bioreactor after each culturing cycle.

In the particular industrial application of plant cell cultures, different well-known culture systems have been used such as stirred tank or airlift reactors. Despite many efforts to commercialize plant metabolites, few achieved commercial success. One reason is the low productivity in spite of the possibility to obtain higher content of desired compound than in whole plant (rosmarinic acid, shikonin, etc.), up to 20 % of dry weight. The main constraint leading to a low productivity remains the low growth rate (below 0.7 day⁻¹, min 20h doubled-time) compared to bacteria. Using batch culture in industrial fermentor means to operate no more than 10-20 runs per year with plant cell cultures in very high cost facilities. It means that the bottleneck for an industrial production is more an economical one than a biological one.

To overcome these problems and decrease production costs, new technologies recently appeared, based on the use of various disposable plastic bags instead of stainless steel fermentor. These new systems using pre-sterile disposable plastic bags are promising because they decrease capital investment since plastic is a low cost material and moreover they eliminate cleaning, sterilization, validation and maintenance of equipment, which is time and cost consuming. It also allows more flexibility in the process, which can be operated by people not skilled in the art that do not need a long training/learning process and/or experience since bags are provided pre-sterile. In addition, using a non-invasive means of agitation allows to minimize mechanical complexity and possibility of contamination. The aeration in such system is done with usual air inlet and bubbling assuming the aeration/agitation and bulk mixing. A reactor can consist of a gas-sparged plastic bag liner in a tank with a headplate that has capabilities for inoculation and media sample removal. Disposable conical plastic bags produced by Osmotec are for small-scale use (few liters), using air bubbles for aeration through an inlet. U.S. Pat. No. 6,432,698 also describes a disposable bioreactor for culturing microorganisms or cells, comprising a gas bubbler, generating gas bubbles for mixing and providing gases, close to airlift bioreactor except it is herein made in plastic material. In these inventions, small bubbles can be detrimental to sensitive cells, increase foam formation and cell to wall adhesion, and/or strip off some useful gazes from the culture medium (ethylene for plant cell for example).

To minimize the cells damage and to improve cell growth and productivity, different bubble-free aeration/mixing systems have been proposed in such disposable apparatus. For example, another suggestion is to use gas permeable plastic bags agitated with a mechanical system or not agitated at all : in U.S. Pat. No. 5,057,429 a gas permeable bag is rotated or shaked to diffuse oxygen and nutrients to the animal cells. A static gas-permeable bag is also described in U.S. Pat. No. 5,225,346. Up to now there is no industrial development of such culture systems mostly because on one hand there is a difficulty to scale-up an external agitation apparatus and on the other hand the problems due to insufficient oxygen supply to the cells in a static bag containing several liters of culture medium. In WO 00/66706, the agitation is done by oscillation about an axis of oscillation of a reactor frame. In the « demande de brevet d'invention FR-A-2519020 », it is also an oscillation about an axis of oscillation which allows the agitation. The axis can be put on the central part of the device or at one of the extremity. Wave Biotech (Singh V, U.S. Pat. No. 6,190,913) has also developed a system using an inflated bag placed on a rocking mechanism that moves the bag inducing a wave-like motion to the liquid contained therein. The rocking mechanism limits the size of the tank because such a mechanical agitation needs complex equipment to reach high volumes of culture; indeed the waves induction mechanism supports the entire culture chamber. In FR-A-2519020, the volume of culture is only at the laboratory scale (2.5 liters) and limited to 200 liters in WO 00/66706. In US 6,190,913, used volumes from 100ml to 500L are claimed. In all these three patents, the entire culture chamber rocks periodically from one side to the other, generating very high pressure on each side which is incompatible with a large scale up.

The aim of the present invention is to provide an efficient and low cost cell culture system via a disposable apparatus, which is easier to scale up than most of disposable culture systems already described.

### Summary of the invention

The present invention relates to a cell culture apparatus according to claim 1.

It also relates to the use of such an apparatus to cultivate plant cells, animal cells, or microorganisms.

### Brief description of the drawings

FIG. 1 is a perspective view of the apparatus.
FIG. 2 is a side view of the apparatus.

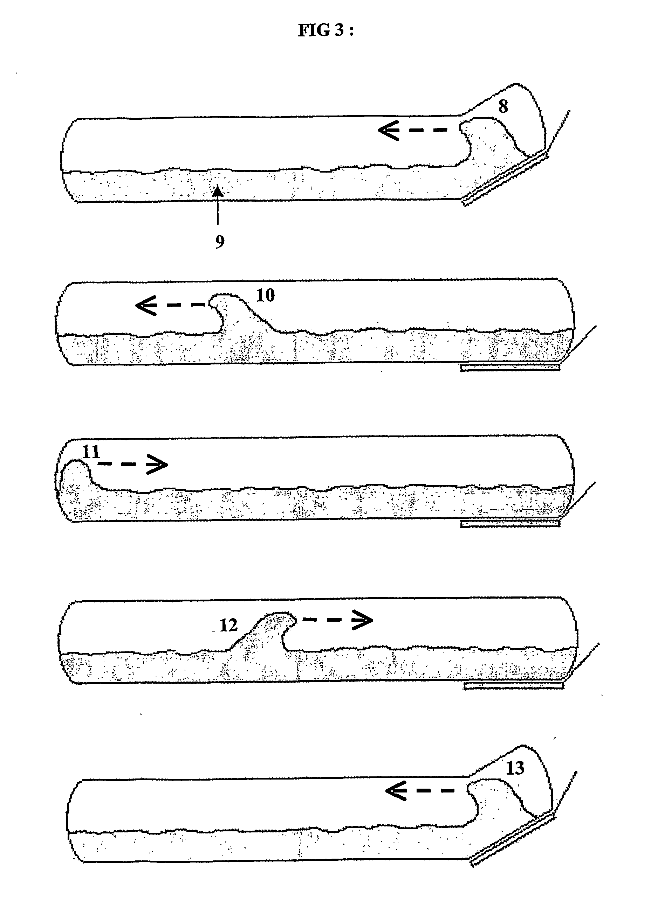

FIG. 3 is schematic representation of waves formation by moving up and down the wave induction mechanism.

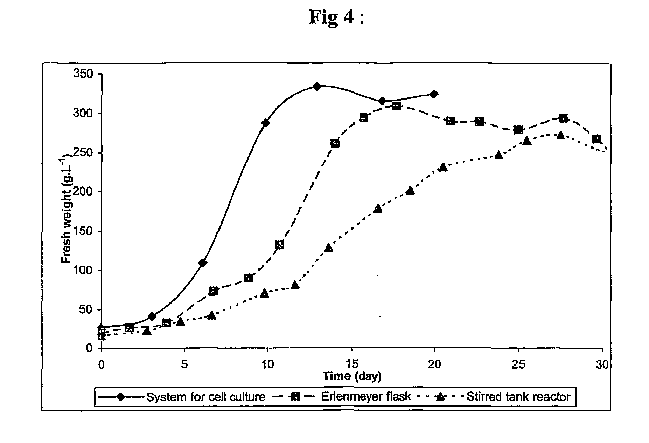

FIG. 4 shows growth kinetics of Soya cells cultured in System for cell culture, Erlenmeyer flask and stirred tank reactor, expressed in g fresh weight per litre.

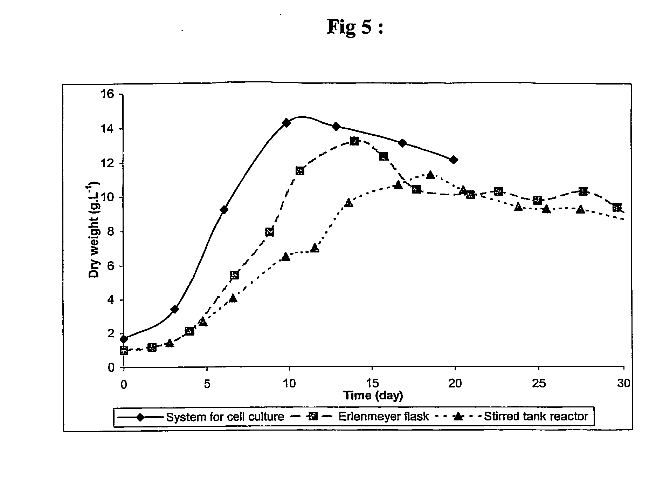

FIG. 5 shows growth kinetics of Soya cells cultured in System for cell culture, Erlenmeyer flask and stirred tank reactor, expressed in g dry weight per litre.

### Detailed description of the invention

The present device provides a simple system for culturing living cells. It consists of two components: a disposable culture chamber and a wave induction mechanism.

An inexpensive, flexible and disposable inflated plastic bag constitutes the culture chamber. This plastic bag is partially filled with liquid cultivation medium and cells, which are mixed and oxygenated owing to a wave movement. In this way, the surface of the medium is continuously renewed and bubble-free aeration can take place that is not detrimental for the cells. Moving up and down at least one extremity of the plastic bag with a simple mechanism causes the induction of the wave movement. This mechanism of wave induction consists of a small plate raised periodically with a motor or a ram linked to a timer. Such a simple waves induction mechanism allows to scale up easily.

As the present invention is disposable and efficient at large scale, it is a good alternative system to decrease production costs in industrial applications. In addition, such a disposable system allows process flexibility and decreases dead time since no cleaning, maintenance or validation is required like in traditional stainless steel devices.

This culture system can be applied for plant cell, animal cell culture or microorganism cultures, with a very easy scale-up from small bag to large one since the wave induction mechanism is very simple. The process allows to produce *de novo* metabolites, biotransformation, recombinant proteins, or to multiply embryogenic plant cell line through batch culture, fed-batch or continuous culture, as well as any other use that could be obvious for the skilled person.

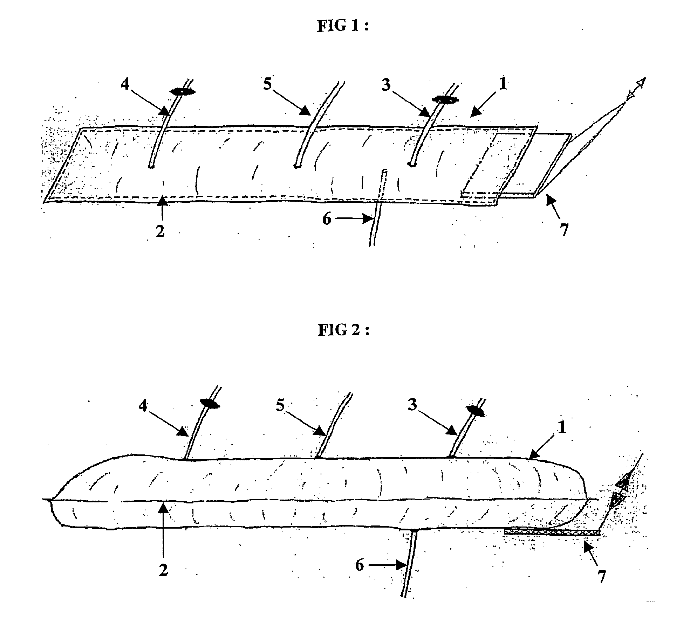

A basic design of the invention is shown in figure 1 (perspective view) and figure 2 (side view). The bioreactor is composed of different parts, comprising at least one culture chamber (1) made of material, such as, for example, flexible biocompatible plastic sheets sealed along their edges (2), to create an interior. Since the present invention is based on the principle of a wave providing agitation, the second essential part is the wave induction mechanism (7) which can consist of a plate placed just under at least one extremity of the culture chamber, for example, or any other appropriate means like a system witch pulls up the extremity of the culture chamber. The wave induction mechanism can also consist in a plate that moves up and down the middle part, or any other part, of the culture chamber.

In a preferred embodiment of the present invention, the culture chamber is a bag made of flexible polypropylene for its sealable and autoclavable properties, so it can be sterilized in a small laboratory autoclave or by any other means well known in the art. However other kinds of plastic materials are also suitable such as polyethylene among others and other kinds of sterilization techniques can be used.

In a preferred embodiment of the invention, flexible biocompatible water proof material are heat-sealed along their edges (2), for example, with a thermic impulse sealer. However other sealing techniques can also be used, in accordance with methods well known in the art including, but not limited to, ultrasound or radio wave welding. Other kinds of plastics that will not require sealing can be manufactured in a different manner such as mold injection for example.

The bag has preferably a pillow shape but other shapes can be used and the dimensions can be varied to suit needs of users. In the present invention, the bag forming the culture chamber may have a square, rectangular, oval, round, or trapezoidal surface. However, rectangular shapes are preferred for an improved efficiency of the method and apparatus of the invention. Particularly preferred dimensions of the bag are 3.20 m length and 0.9 m width for 100 liters of culture or 3 m of length and 0.3 m of width for 25 liters. However, length can go up to 10 m and over, and the present invention allows to have from 1 liter to several hundred liters, such as 100, 300, 500, 1000 L or more culture medium.

In the preferred embodiment, at least two tubes, connected to the upper side of the culture chamber, can ensure oxygen renewal ; the first (3) one provides the air intake (compressed air or other gases according to the needs of the culture) and the second one vents the exhaust gases (4). These two tubes are equipped, in the most preferred embodiment, with filters to prevent airborne contamination, for example 0.22 µm filters. An other means to provide oxygen supply and elimination of gases produced by the culture, consists in providing, on a at least part of the upper surface of the bag, a gas permeable membrane. Such membrane must have a permeability to gas and a surface sufficient to assure a good oxygenation of the culture. The membrane can for example be sealed or welded on the bag. These membranes must preferably be impervious to liquid, water vapor and contaminants.

In addition, in the basic design shown in figure 1, at least two other tubes can be connected to the culture chamber. One (5) is connected on the superior side of the bag, to fill the bioreactor with an inoculum suspended in fresh sterilized culture medium. The other one (6) is connected on the inferior side of the bag to draw samples or to harvest the culture. These tubes are preferably made of (but not limited to) autoclavable silicone with a ratio inner diameter/outer diameter of 8/12 mm but this ratio can of course be changed to suit needs of the operator.

The second essential part of the culture system is the wave induction mechanism (7). As shown in figure 2, this mechanism is made amongst other things of a small plate and placed preferably just under at least one extremity of the culture chamber, but said plate can be placed under the middle point of the culture chamber, or under any other part of said culture chamber. In a preferred embodiment, the width of the plate is equal to the width of the culture chamber and the length of the plate is between 5 and 50%, more preferably 8 to 20 % of the length of the culture chamber. Moving up and down through an angle of 1° to 90°, preferably 1 to 45°, most preferably 1° to 25°, the small plate induces a wave (8) into the liquid culture (9) that provides both mixing and oxygenation of the culture. Since the bag is inflated, it allows the wave to propagate along the culture chamber (10). In a preferred embodiment, the height of the culture medium is 10 to 30 % of the height of the inflated culture chamber. Surprisingly when the wave arrives at the opposed extremity (11), it returns at the initial point (12) without breaking. Then, the mechanism creates an impulse again (13). To improve the return of the wave, it is also possible to slightly raise the opposed extremity.

The small plate of the wave induction mechanism can be raised periodically with a motorized arm or with a ram but other kinds of equipment can be chosen. This equipment is preferably connected to a timer, in order to choose the more appropriate frequency of wave induction.

When a very long culture chamber is used for large volume of culture, for example over hundred liters, it is also possible to place a wave induction mechanism at each extremity of the culture chamber. In this case, the wave induction mechanism lifts up alternatively between 5 and 50% or more preferably between 7 and 20% of the length of the culture chamber.

The present invention is based on the fact that waves induction provides liquid culture mixing and bubble-free aeration. The oxygen transfer is done by diffusion from the headspace air to the liquid culture and provides growth without shear stress.

This culture system is easy to operate since both culture chamber and the means of agitation are simple. Scale up is also easy to achieved from small volumes to several hundred liters since the waves induction mechanism doesn't support the entire culture chamber but have only to raise at least one extremity of the culture chamber.

Preferably, the culture chamber is filled with the cells and culture medium at a filling rate of 20 to 80%, most preferably 20 to 40%.

### Example:

The following example is illustrative of some of the products and methods of making the same falling within the scope of the present invention. It is not to be considered in any way limitative of the invention. Changes and modifications can be made with respect to the invention. That is, the skilled person will recognise many variations in these examples to cover a wide range of formulas, ingredients, processing, and mixtures to rationally adjust the naturally occurring levels of the compounds of the invention for a variety of applications.

### Example: Comparison of growth with Soya cell cultures

The ability of the invention to grow Soya cells has been demonstrated using batch cultures. This is comparable or better than in Erlenmeyer flask or stirred tank bioreactor, even at larger scale.

Tissue culture strains of *Glycine max* (L.) Merr. were initiated from different cultivars on Gamborg et al. medium (1968) supplemented with 20g.L⁻¹ sucrose, 7g.L⁻¹ agar (bacto-agar Difco) and 1mg.L⁻¹ 2,4-Dichlorophenoxyacetic acid. The pH is adjusted to 5.8 prior autoclaving (30 min at 115°C). One strain (13406, cv. Maple arrow) was transferred in liquid medium (same medium as for tissue cultures without agar and 30g.L⁻¹ sucrose) and subcultured in 250mL Erlenmeyer flask (3g.L⁻¹ fresh weight with 100mL medium) every two weeks, in the same conditions than tissue culture collection. The Erlenmeyer flasks are placed on an orbital shaker at 100 rpm (shaking diameter 20mm).

A 14L stirred tank bioreactor (New Brunswick Scientific) with two six flat blade impellers, is used with the same medium and conditions of temperature and pH as mentioned above. The bioreactor containing 9L of fresh medium is autoclaved 40min at 115°C. Fourteen day old Soya cells are filtered from two 1L Erlenmeyer flasks (500ml medium). 300g fresh weight was put into 1L of fresh medium in a sterile tank with a specific output to be connected aseptically to the bioreactor for inoculation. The stirrer speed is adjusted at 100rpm. Dissolved oxygen is maintained at 30% by increasing or decreasing air flow rate, using a biocontroller equipped with a sterilizable oxygen probe (Ingold), and a mass flowmeter.

A system for cell culture (as previously described) is filled with 10 L of Soya cells in fresh culture medium (30 g/L fresh weight). Temperature of the room is regulated at 25 °C and a wave is generated every 4 seconds (by programming the waves induction mechanism as mentioned above).

Growth measurements: Samples of cultivation bulk are taken at some specific periods of growth from flasks, stirred tank bioreactor and system for cell culture and sample volume is measured. Cells are then removed from liquid culture via filtration and weighted (fresh weight). An aliquot of this biomass is put into a drying room at 100°C during 24 hours and then weighed precisely (dry weight).

This example shows that the 10L scale system for cell culture seems to provide a gentle environment to the cells, comparable with flasks and better than the stirred tank reactor. Cell damages are limited and mass and gas transfers are efficient in the operated conditions.

As already mentioned above, the present invention provides numerous advantages, which in turn are keys to economic benefits:

It provides a gentle environment to grow plant cells
Scale-up is easy
It is disposable
It is easy to operate

## Claims

1. Cell culture apparatus comprising
- a flexible culture chamber, and
- a wave induction mechanism, comprising a small plate raised periodically,
wherein the wave induction mechanism moves from 8 to 20% of the surface area of the lower part of the culture chamber, and wherein the culture chamber is a flexible plastic bag.

2. Cell culture apparatus according to claim 1 or claim 2, wherein the wave induction mechanism moves part of the lower part of the culture chamber to an angle of 1 to 90°, preferably 1 to 25°.

3. Cell culture apparatus according to any preceding claim wherein the culture chamber comprises means to circulate the air such as tube or air permeable membrane.

4. Cell culture apparatus according to any preceding claim wherein the wave induction mechanism lifts up alternatively between 5 and 50% of the length of the culture chamber.

5. Cell culture apparatus according to any preceding claim wherein the culture chamber filling rate is from 10 to 80%, preferably 20-40%.

6. Use of a cell culture apparatus according to any preceding claims to cultivate plant cells, animal cells, or microorganisms.

7. Use of a cell culture apparatus according to any preceding claim wherein cells are producing biomass cells, embryogenic plant cells, metabolites, secondary plant metabolites and / or recombinant molecules

## Patentansprüche

1. Zellkulturvorrichtung, enthaltend
- eine flexible Kulturkammer, und
- einen Welleninduktionsmechanismus, der eine kleine Platte umfasst, die periodisch aufgerichtet wird, wobei der Welleninduktionsmechanismus von 8 bis 20% der Fläche des unteren Anteils der der Kulturkammer bewegt, und wobei die Kulturkammer eine flexible Plastiktüte ist.

2. Zellkulturvorrichtung nach Anspruch 1 oder Anspruch 2, wobei der Welleninduktionsmechanismus einen Teil des unteren Anteils der Kulturkammer zu einem Winkel von 1 bis 90°, vorzugsweise 1 bis 25° bewegt.

3. Zellkulturvorrichtung nach einem der vorhergehenden Ansprüche, wobei die Kulturkammer Hilfsmittel wie beispielsweise Röhrchen oder luftdurchlässige Membran umfasst, um die Luft zur zirkulieren.

4. Zellkulturvorrichtung nach einem der vorhergehenden Ansprüche, wobei der Welleninduktionsmechanismus alternativ zwischen 5 und 50% der Länge der Kulturkammer anhebt.

5. Zellkulturvorrichtung nach einem der vorhergehenden Ansprüche, wobei die Kulturkammerfüllungsrate von 10 bis 80%, vorzugsweise 20 - 40% ist.

6. Verwendung einer Zellkulturvorrichtung nach einem der vorhergehenden Ansprüche zur Kultivierung von Pflanzenzellen, Tierzellen oder Mikroorganismen.

7. Verwendung einer Zellkulturvorrichtung nach einem der vorhergehenden Ansprüche, wobei Zellen Biomassezellen, embryogene Pflanzenzellen, Metabolite, sekundäre Pflanzenmetabolite und/oder rekombinante Moleküle produzieren.

## Revendications

1. Dispositif de culture cellulaire comprenant :
- une chambre de culture flexible, et
- un mécanisme d'induction de vague, comprenant une petite plaque qui est élevée périodiquement,
dans lequel le mécanisme d'induction de vague déplace de 8 à 20 % de la surface de la partie inférieure de la chambre de culture, et
dans lequel la chambre de culture est un sachet plastique flexible.

2. Dispositif de culture cellulaire selon la revendication 1 ou la revendication 2, dans lequel le mécanisme d'induction de vague déplace une partie de la partie inférieure de la chambre de culture à un angle de 1 à 90°, de préférence de 1 à 25°.

3. Dispositif de culture cellulaire selon l'une quelconque des revendications précédentes, dans lequel la chambre de culture comprend des moyens pour faire circuler de l'air tels qu'un tube ou une membrane perméable à l'air.

4. Dispositif de culture cellulaire selon l'une quelconque des revendications précédentes, dans lequel le mécanisme d'induction de vague soulève alternativement entre 5 et 50 % de la longueur de la chambre de culture.

5. Dispositif de culture cellulaire selon l'une quelconque des revendications précédentes, dans lequel le taux de remplissage de la chambre de culture est compris entre 10 et 80 %, de préférence 20-40 %.

6. Utilisation d'un dispositif de culture cellulaire selon l'une quelconque des revendications précédentes afin de cultiver des cellules végétales, des cellules animales, ou des micro-organismes.

7. Utilisation d'un dispositif de culture cellulaire selon l'une quelconque des revendications précédentes, dans lequel des cellules produisent des cellules de biomasse, des cellules végétales embryogènes, des métabolites, des métabolites végétaux secondaires et / ou des molécules recombinées.
